# EUROPEAN PATENT APPLICATION

(11) **EP 2 368 488 A1**
(43) Date of publication of application: **28.09.2011**
(21) Application number: 11152588.7
(22) Date of filing: 28.01.2011
(51) Int. Cl.: A61B 1/04

(54) **Endoscope system comprising calibration means and calibration method thereof**

(30) Priority: 25.03.2010 JP 2010070192
(71) Applicant: Fujifilm Corporation, Minato-ku Tokyo (JP)
(72) Inventor: MATSUBARA, Kenta, Kanagawa (JP)
(74) Representative: Klunker . Schmitt-Nilson . Hirsch

(57) **Abstract**

An electronic endoscope system includes a light source device for emitting light and an electronic endoscope having a phosphor for producing white light with which a subject tissue in a body cavity is illuminated by causing the light to hit the phosphor as excitation light, and an image sensor for outputting image data of an image obtained by photoelectric conversion of reflected light of the white light illuminating the subject tissue, a wavelength detector for detecting a wavelength of the light, a calculator for calculating a wavelength shift amount which is a difference between a wavelength detected and a preset reference wavelength, a first correction unit for correcting a light amount of the light according to the calculated wavelength shift amount, and a second correction unit for correcting the gain in photoelectric conversion by the image sensor.

## Description

### BACKGROUND OF INVENTION

The present invention relates to an endoscope system comprising an endoscope light source and an endoscope including calibration means and a calibration method thereof.

In recent years, a number of diagnoses and treatments using electronic endoscopes have been made in the field of medicine. A typical electronic endoscope is equipped with an elongated insertion section that is inserted into a subject's body cavity. The insertion section has therein incorporated an imager such as a CCD at the tip thereof. The electronic endoscope is connected to a light source device, which emits light from the tip of the insertion section to illuminate the inside of a body cavity. With the inside of the body cavity illuminated by light, the subject tissue inside the body cavity is imaged by an imager provided at the tip of the insertion section. Images acquired by imaging undergoes various kinds of processing by a processor connected to the electronic endoscope before being displayed by a monitor. Thus, the electronic endoscope permits real-time observation of images showing the inside of the subject's body cavity and thus enables sure diagnoses.

The light source device uses a white light source, such as a xenon lamp and a white LED, capable of emitting white broadband light whose wavelength ranges from a blue region to a red region. Use of white broadband light to illuminate the inside of a body cavity permits observing the whole subject tissue from the acquired images thereof.

In observation of acquired images, the actual color tone of an imaged site inside a biological body and a color tone of an acquired image displayed on an image monitor may vary depending on transmission characteristics of the endoscope, fluorescence characteristics of the phosphor, reflection characteristics of the subject tissue and the spectral sensitivity of the image sensor, and this may greatly affect the recognition and diagnosis of lesions.

The methods proposed in JP 2008-36035 A and JP 2008-93225 A are among known solutions to this problem.

JP 2008-36035 A, for example, comprises a band-limited normal illumination production means for producing discrete band-limited normal illumination light obtained by limiting the visible RGB wavelength band of light to a bandwidth having a given light amount to acquire an image of a given color tone by reducing the effects produced by the spectral sensitivity of the image sensor when observation is made using normal illumination light.

JP 2008-93225 performs color reproduction of an acquired image of a site inside a biological body illuminated by a first illumination means of an endoscope under illumination provided by a second illumination means based on the characteristics information given by the first illumination means, the second illumination means providing image observation environment, an imaging means for acquiring the image, and a display device for displaying the image and on the spectral reflectance at a plurality of points in the subject biological body to enable examinations and treatments under conditions closer to observation by the naked eye.

### SUMMARY OF INVENTIION

Thus, while a xenon lamp or the like is used as a white light source, a compact white phosphor using laser light as excitation light is also on the way to practical use to produce white illumination light providing yet higher luminance.

The problems encountered in the use of laser are the individual variations in incoming wavelength and light amount and the variations produced in manufacture, and variations that occur as time passes. Other problems include variations in fluorescence characteristics caused by the variation in incoming light and the variation in characteristics of the phosphor itself. These variations affect, in particular, the color reproducibility and resolution of images. Further, laser causes a specific phenomenon that the wavelength is shifted by the operating current. Therefore, there is a demand for a system allowing easy and high-accuracy detection of information on variations of the incoming wavelength and the light amount to be made at the factory and on the market and permitting feedback thereof to the system.

Endoscope systems typically comprise a laser light source for emitting narrowband light and an endoscope that includes a phosphor for producing a multicolor wavelength.

On the market, an n number of light source devices are used with an m number of endoscopes, that is, one particular light source device is not necessarily used with one particular endoscope. Therefore, light source devices and endoscopes must be usable in any combination thereof.

An object of the invention is to provide an endoscope system whose color reproducibility and image resolution can be made free from the effects of the variations in the light source device and the endoscope and the effects produced when a plurality of light source device and endoscope are used in various combinations by calibration performed using a compact and inexpensive measuring means.

In order to achieve the above objectives, a first aspect of the present invention provides an electronic endoscope system, comprising: a light source device for emitting light having a given wavelength band, an electronic endoscope comprising a phosphor for producing white light with which a subject tissue in a body cavity is illuminated by causing the emitted light from the light source device to hit the phosphor as excitation light, and an image sensor for outputting image data of an image obtained by photoelectric conversion of reflected light of the white light illuminating the subject tissue, wavelength detection means for detecting a wavelength of the emitted light from the light source device, wavelength shift amount calculation means for calculating a wavelength shift amount, which is a difference between a wavelength detected by the wavelength detection means and a preset reference wavelength, and first correction means for correcting a light amount of the emitted light from the light source device according to the wavelength shift amount calculated by the wavelength shift amount calculation means and second correction means for correcting a gain in the photoelectric conversion by the image sensor.

Preferably, the first correction means comprises a first reference table representing a relationship between a wavelength of light and an integrated value of a sensitivity of the image sensor and a reflectance of the reflected light, obtains an integrated value corresponding to a wavelength of the emitted light from the light source device referring to the first reference table, and changes the light amount correction amount according to the obtained integrated value.

Preferably, the first correction means comprises plural first reference tables each representing a relationship between a wavelength of light and an integrated value of a sensitivity of the image sensor and a reflectance of the reflected light for each site or disease imaged by the electronic endoscope, selects one first reference table from the plural first reference tables according to the site or disease to be imaged by the electronic endoscope, obtains an integrated value corresponding to a wavelength of the emitted light from the light source device referring to the selected one first reference table, and changes the light amount correction amount according to the obtained integrated value.

Preferably, the light source device is a laser light source for emitting laser light, and the wavelength shift amount calculation means calculates the wavelength shift amount by further adding thereto a wavelength shift amount of the laser light that changes according to an operating current value of the laser light source.

Preferably, the wavelength detection means obtains a transmittance being a ratio between a first light amount and a second light amount, from the first light amount of the emitted light from the light source device and the second light amount of the emitted light from the light source device and passed through a slope type dichroic filter whose transmittance changes for light having a given wavelength in proportion to the wavelength of the light, and detects the wavelength of the emitted light from the light source device according to the obtained transmittance.

Preferably, the second correction means comprises a second reference table representing a relationship between a wavelength shift amount and a variation amount in a pixel value in the image data obtained through the photoelectric conversion by the image sensor, obtains the variation amount of the pixel value in the image data corresponding to the wavelength shift amount calculated by the wavelength shift amount calculation means referring to the second reference table, corrects a pixel value in the image data for a case where the emitted light from the light source device is photoelectrically converted according to the obtained variation amount of the pixel value in the image data, and further corrects the gain in the photoelectric conversion by the image sensor so that the corrected pixel value of the image data agrees with a pixel value in image data for a case where the light having the reference wavelength is photoelectrically converted.

In order to achieve the above objectives, a second aspect of the present invention provides a method of calibrating an electronic endoscope provided with a light source device for emitting light having a given wavelength band and an image sensor that illuminates a subject tissue in a body cavity with white light produced by causing excitation light emitted from the light source device to hit a phosphor and outputs image data obtained by photoelectric conversion of reflected light of the white light illuminating the subject tissue, the method comprising: a wavelength detection step of detecting a wavelength of light emitted from the light source device, a wavelength shift amount detection step of detecting a wavelength shift amount, which is a difference between the detected wavelength and a preset reference wavelength, a first correction step of correcting a light amount of the light emitted from the light source device according to the calculated wavelength shift amount, and a second correction step of correcting a gain in photoelectric conversion by the image sensor according to the calculated wavelength shift amount.

Preferably, the first correction step comprises obtaining an integrated value corresponding to a wavelength of the emitted light from the light source device referring to a first reference table representing a relationship between a wavelength of light and an integrated value of a sensitivity of the image sensor and a reflectance of the reflected light, and changing the light amount correction amount according to the obtained integrated value.

Preferably, the first correction step comprises selecting one first reference table according to a site or disease to be imaged by the electronic endoscope from plural first reference tables each representing a relationship between a wavelength of light and an integrated value of a sensitivity of the image sensor and a reflectance of the reflected light for each site or disease imaged by the electronic endoscope, obtaining an integrated value corresponding to a wavelength of the emitted light from the light source device referring to the selected one first reference table, and changing the light amount correction amount according to the obtained integrated value.

Preferably, the light source device is a laser light source for emitting laser light, and the the wavelength shift amount calculation step calculates the wavelength shift amount by further adding thereto a wavelength shift amount of the laser light that changes according to an operating current value of the laser light source.

Preferably, the calibration method further comprises: a detecting step of detecting a first light amount of the emitted light from the light source device and a second light amount of the emitted light from the light source device and passed through a slope type dichroic filter whose transmittance changes for light having a given wavelength in proportion to the wavelength of the light, and the wavelength detection step comprising obtaining a transmittance being a ratio between the first light amount and the second light amount, from the obtained first and the second light amounts, and detecting the wavelength of the emitted light from the light source device according to the obtained transmittance.

Preferably, the second correction step comprises obtaining a variation amount of a pixel value in image data corresponding to the calculated wavelength shift amount referring to a second reference table representing a relationship between a wavelength shift amount and a variation amount in a pixel value in the image data obtained through the photoelectric conversion by the image sensor, correcting a pixel value in the image data for a case where the emitted light from the light source device is photoelectrically converted according to the obtained variation amount of the pixel value in the image data, and further correcting the gain in the photoelectric conversion by the image sensor so that the corrected pixel value of the image data agrees with a pixel value of the image data for a case where the light having the reference wavelength is photoelectrically converted.

Preferably, the calibration method further comprises: a step of illuminating a reference chart with light having the reference wavelength, and a step of obtaining the image data outputted from the image sensor after the photoelectric conversion of the reflected light from the reference chart by the image sensor, and the second correction step comprises adjusting the gain in the photoelectric conversion by the image sensor so that a pixel value in the image data outputted by the image sensor after the photoelectric conversion of the reflected light from the reference chart agrees with a reference value corresponding to the reference chart.

According to the present invention, in an endoscope system using laser light as light source, even when there are variations in incoming wavelengths and light amount, transmission characteristics of the endoscope, fluorescence characteristics of the phosphor, reflection characteristics of subject tissues, and spectral sensitivity characteristics of the image sensor, in both the light source device and the endoscope, the color reproducibility and image resolution in particular can be made free from the effects of these variations by performing calibration as appropriate according to the invention in one of the light source device and the endoscope, or in each of the combinations of the light source device and the endoscope.

The color reproducibility and image resolution can also be made free from the effects produced by a wavelength shift caused by the operating current of the laser and from the effects that may be produced when the endoscope system is used in any of the combinations of light sources and endoscopes.

Further, according to the present invention, calibration may be performed using a compact and inexpensive measuring means without the need to use large and expensive measuring instruments such as spectral analyzers as was conventionally the case.

### BRIEF DESCRIPTION OF DRAWINGS

Fig. 1 is an external view of an electronic endoscope system according to an embodiment of the invention.
Fig. 2 is a block diagram illustrating an electric configuration of the electronic endoscope system of Fig. 1.
Fig. 3 is a graph illustrating a relationship between a transmittance of a slope type dichroic filter of the invention and a wavelength of first narrowband light, incoming light.
Fig. 4 is a graph illustrating a relationship between a transmittance of a slope type dichroic filter of the invention and a wavelength of incoming light, second narrowband light.
Fig. 5 is a graph illustrating actual filter characteristics of the slope type dichroic filter of the invention.
Fig. 6 is a graph illustrating a relationship between shift amounts of the first and the second narrowband light of the invention and a reflectance of a biological body (stomach) and a spectral sensitivity of an image sensor (CCD).
Fig. 7 is a graph illustrating spectral transmittances of red, green, and blue filters.
Fig. 8 is a view for explaining imaging operations of an image sensor (CCD).
Fig. 9 is a block diagram of a method of calibrating a light source device of the invention.
Fig. 10 is a block diagram of a method of calibrating an endoscope of the invention.
Fig. 11 is a block diagram of a method of calibrating the endoscope system of the invention practiced in the market.

### DETAILED DESCRIPTION OF INVENTION

First, the electronic endoscope system according to an embodiment of the invention will be described.

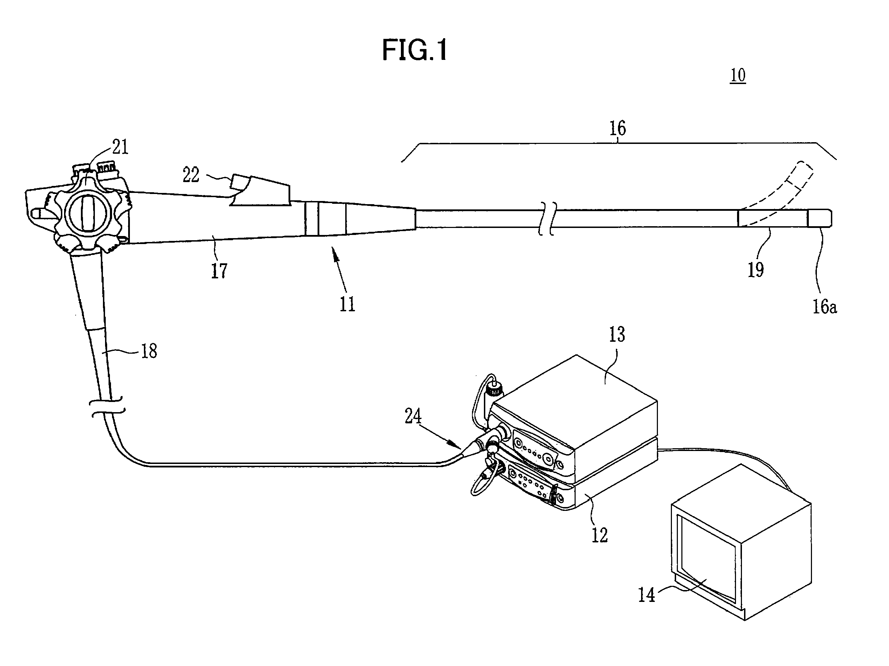

As illustrated in Fig. 1, an electronic endoscope system 10 comprises an endoscope (endoscope device) 11 for imaging the inside of a subject's body cavity, a processor 12 for producing an image of a subject tissue in the body cavity according to a signal acquired by imaging, a light source device 13 for supplying light for illuminating the inside of the body cavity, and a monitor 14 for displaying the image of the inside of the body cavity. The endoscope 11 comprises a flexible insertion section 16 that is inserted into a body cavity, an operating section 17 provided at the base of the insertion section 16, and a universal cord 18 for connecting the operating section 17 to the processor 12 and the light source device 13.

The insertion section 16 has a bending portion 19 at the tip thereof comprising connected bending pieces. The bending portion 19 bends up and down, left and right in response to the operation of an angle knob 21 of the operating section 17. The bending portion 19 has at its tip a leading end portion 16a incorporating an optical system and other components for imaging the inside of a body cavity. The leading end portion 16a can be directed in a desired direction in the body cavity according to a bending operation of the bending portion 19.

The universal cord 18 has a connector 24 provided on the side thereof leading to the processor 12 and the light source device 13. The connector 24 is a composite type connector composed of a communication connector and a light source connector and removably connects the endoscope 11 to the processor 12 and the light source device 13 through the connector 24.

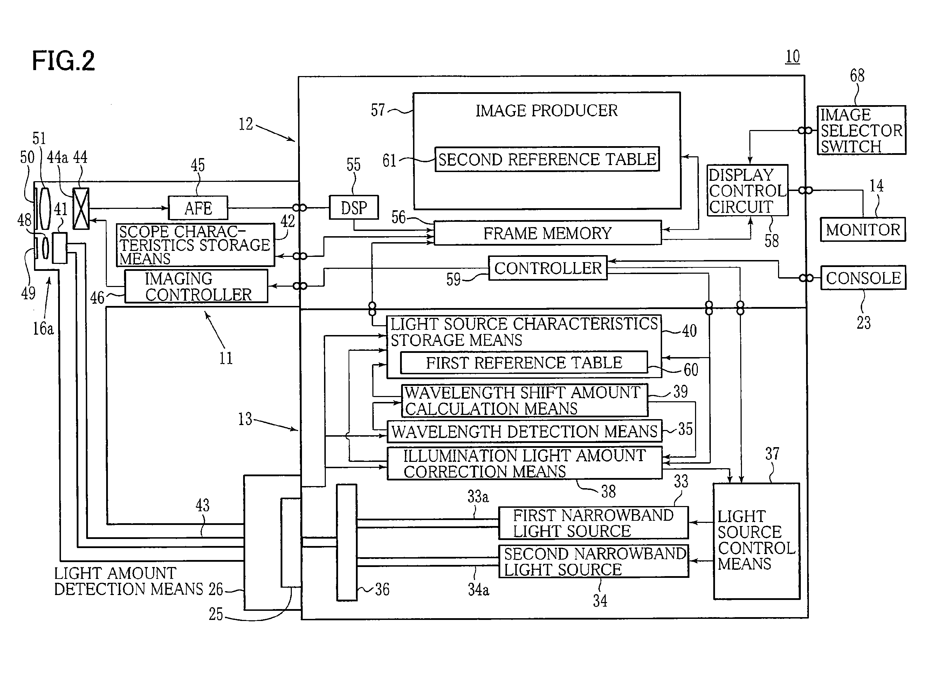

As illustrated in Fig. 2, the light source device 13 comprises first and second narrowband light sources 33, 34, a coupler 36, a light source control means 37, an illumination light amount correction means 38, a wavelength detection means 35, a wavelength shift amount calculation means 39, and a light source characteristics storage means 40. The figure also illustrates a slope type dichroic filter 25 used for calibration made by the electronic endoscope system 10 and a light amount detection means 26.

The first and the second narrowband light sources 33, 34 are laser diodes or the like. The first narrowband light source 33 produces narrowband light having a wavelength limited to 400 nm +/- 10 nm, preferably 405 nm (referred to below as "first narrowband light N1"), and the second narrowband light source 34 produces narrowband light having a wavelength limited to 440 nm +/- 10 nm, preferably 445 nm (referred to below as "second narrowband light N2"). The first and the second narrowband light sources 33, 34 are connected respectively to first and the second narrowband optical fibers 33a, 34a, allowing the first and the second narrowband light N1 and N2 emitted by their respective light sources to enter the first and the second narrowband optical fibers 33a, 34a.

The first narrowband light N1 passes through a phosphor 41 and is emitted therefrom as it is. The second narrowband light N2 also acts as excitation light, causing the phosphor 41 to emit broadband light BB, white light, while a given amount of the second narrowband light N2 is also emitted therefrom.

The phosphor 41 used herein may, among others, be Micro White (trademark) provided by Nichia Corporation, for example.

The second narrowband light N2 emitted from the second light source device 34 of the light source device 13 is caused to hit the phosphor 41 as excitation light, whereupon the phosphor 41 produces broadband light BB having a wavelength ranging from a blue region to a red region (about 470 nm to 700 nm).

The coupler 36 connects a light guide 43 in the endoscope to the first and the second narrowband optical fibers 33a, 34a. Thus, the first and the second narrowband light N1, N2 can enter the light guide 43 through the first and the second narrowband optical fibers 33a, 34a.

When the calibration of the electronic endoscope system 10 is performed, the slope type dichroic filter 25 is disposed close to an outlet of the light source device 13 from which the light guide 43 extends to measure the wavelengths of the light emitted from the first and the second narrowband light sources 33, 34.

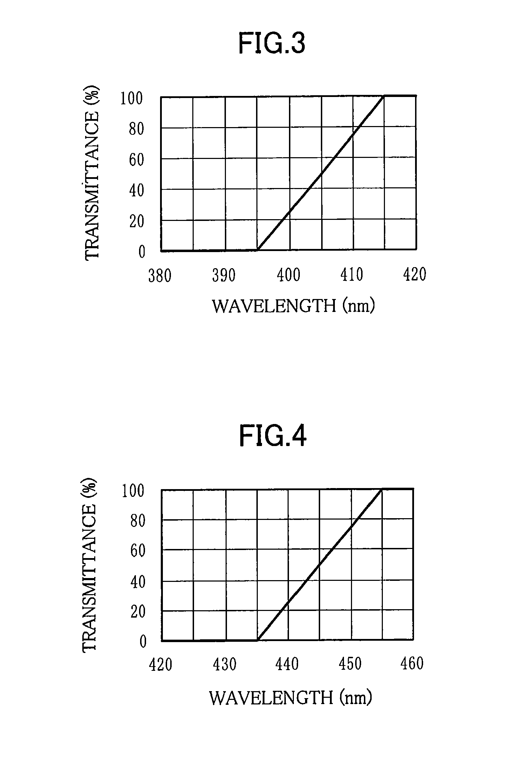

The slope type dichroic filter 25 changes the transmittance of light in proportion to the wavelength for light having a given wavelength band. For example, when a wavelength of the first narrowband light N1 (e.g. 405 nm) is to be detected, a slope type dichroic filter is used whose transmittance changes from 0% to 100% in proportion to the wavelength of 395 nm to 415 nm as illustrated in Fig. 3 for light having a wavelength of 395 nm to 415 nm. When a wavelength of the second narrowband light N2 (e.g. 445 nm) is to be detected, a slope type dichroic filter is used whose transmittance changes from 0% to 100% in proportion to the wavelength of 435 nm to 455 nm as illustrated in Fig. 4 for light having a wavelength of 435 nm to 455 nm.

Because the transmittance of the slope type dichroic filter 25 changes according to the wavelength of the light passing through it, the wavelength of the illumination light can be obtained from the transmittance characteristics of the installed slope type dichroic filter 25 by obtaining the light amount ratio (i.e. transmittance) between a case where the slope type dichroic filter 25 is installed and another case where it is not installed.

The slope type dichroic filters 25 are switched between them so as to meet the wavelengths of the first and the second narrowband light N1, N2.

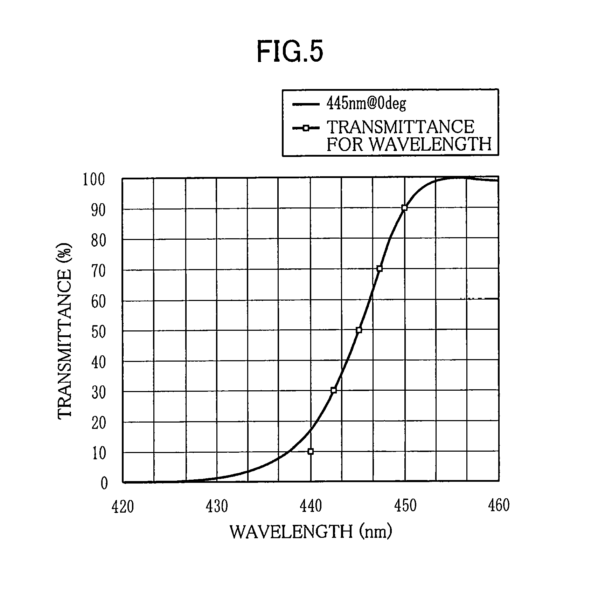

Figs. 3 and 4 illustrate ideal characteristics of the slope type dichroic filter 25; the graph in Fig. 5 illustrates actual characteristics thereof.

Fig. 5 illustrates actual characteristics of the slope type dichroic filter 25 corresponding to the light having a wavelength band of 435 nm to 455 nm illustrated in Fig. 4. From Fig. 5, it appears that the slope type dichroic filter 25 shows substantially linear characteristics in the vicinity of 445 nm.

Also in a filter characteristics region of the slope type dichroic filter 25 outside the linear characteristics region, the wavelength of illumination light of interest can be obtained from the transmittance characteristics by previously measuring initial characteristics of the slope type dichroic filter 25, storing the initial characteristics data in the light source characteristics storage 40 or the like, and correcting the obtained measurements based on the initial characteristics data when measuring the wavelength.

When the electronic endoscope system 10 is calibrated, the light amount detection means 26 is installed close to the outlet of the light source device 13 from which the light guide 43 extends or immediately behind the slope type dichroic filter 25 when the slope type dichroic filter 25 is installed to measure the illumination light amount of the passing illumination light.

The measured illumination light amount is sent to the illumination light amount correction means 38 and the wavelength detection means 35 as illumination light amount data.

The wavelength detection means 35 obtains the transmittance, i.e., the ratio between a first and a second light amount, from the light amount of the light emitted from the light source device 13 (first light amount) and the light amount of the light emitted from the light source device 13 and passed through the slope type dichroic filter 25 (second light amount), both detected by the light amount detection means 26 for both the first and the second narrowband light N1, N2, and detects the wavelength of the light emitted from the light source device 13 according to the obtained transmittance. The detected wavelength is sent to a wavelength shift amount calculation means as wavelength data.

The wavelength shift amount calculation means 39 calculates a wavelength shift amount that is the difference between a wavelength detected by the wavelength detection means 35 and a previously set reference wavelength (e.g. 405 nm for the first narrowband light N1 and 445 nm for the second narrowband light N2). The calculated wavelength shift amount is sent to the illumination light amount correction means 38 as wavelength shift amount data.

The illumination light amount correction means 38 corrects the light amounts of the first and the second narrowband light N1, N2 emitted from the light source device 13 according to the wavelength shift amounts calculated by the wavelength shift amount calculation means 39, that is, so as to counteract the effects by the wavelength shift amounts.

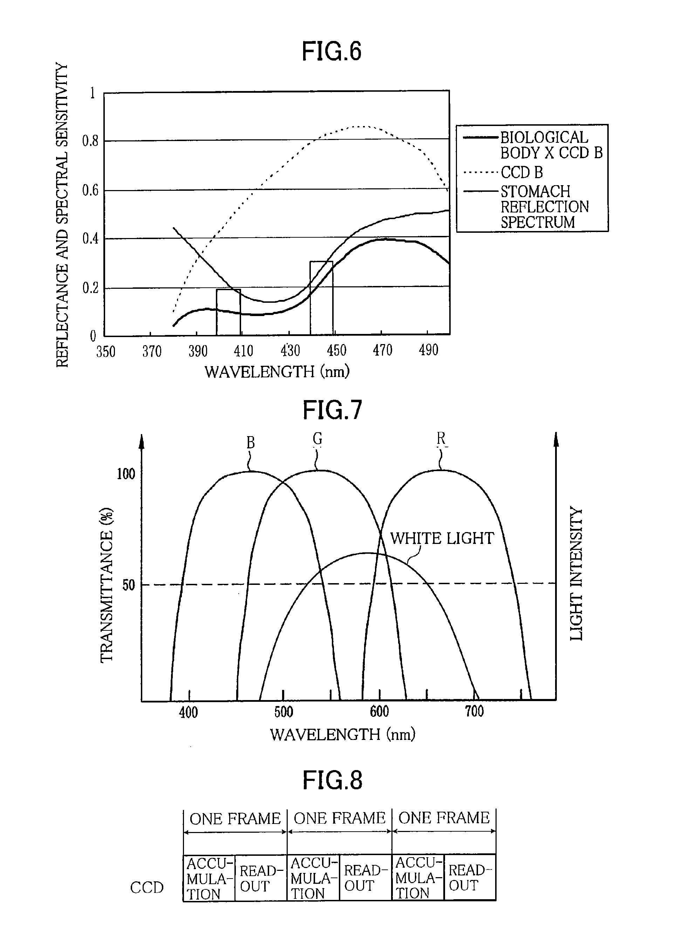

Fig. 6 is a graph illustrating a relationship between the wavelength of light and the spectral sensitivity of the B color of the CCD 44 and the reflectance of the reflected light from the stomach. The horizontal axis of the graph in that figure shows the spectral sensitivity of the B color of the CCD 44; the vertical axis shows the reflectance of the reflected light from the stomach. As illustrated in that figure, the integrated value (shown in thick line) of the spectral sensitivity of the B color of the CCD 44 (shown in dotted line) and the reflectance of the reflected light from the stomach (shown in solid line), for example, changes with the wavelength of the light. In the case shown in Fig. 6, the integrated value is greater with the wavelength 445 nm of the second narrowband light N2 than with the wavelength 405 nm of the first narrowband light N1. That is, the effects produced on the pixel values in image data obtained through photoelectric conversion by CCD 44 vary with the wavelength of the light emitted from the light source device. Accordingly, the effects produced on the pixel values in image data vary with the wavelength of the light even with the same wavelength shift amount, and therefore the illumination light amount correction means 38 preferably changes the correction amount for correcting the light amount of the light emitted from the light source device 13 according to the wavelength of the light.

The illumination light amount correction means 38 contains a first reference table 60 representing a relationship between, for example, the wavelength of the light as shown in thick line in Fig. 6 and the integrated value of the sensitivity of the CCD 44 and the reflectance of the reflected light from a subject tissue. The illumination light amount correction means 38 obtains the integrated value corresponding to the light emitted from the light source device 13 by referring to the first reference table 60 and changes (sets) the light amount correction amount according to the obtained integrated value. The first reference table 60 may alternatively be stored in the light amount characteristics storage means 40.

In addition to the first reference table 60, the light source characteristics storage means 40 have previously stored therein given reference light amount data used for light source calibration, the transmittance characteristics data of the slope type dichroic filter 25, the transmittance characteristics data of a reference scope, and the fluorescence characteristics data of a reference phosphor, and stores light source wavelength data and, when the reference scope is installed, the light amount data for the first and the second narrowband light sources 33, 34 measured in the light source calibration.

The reference light amount is a light amount providing a reference for adjusting the illumination light amount from the light source in the light source calibration; the reference scope is an ideal endoscope provided with reference transmittance characteristics and a reference phosphor; and the reference phosphor is an ideal phosphor having reference fluorescence characteristics (characteristics of emitting given broadband light when irradiated by a given excitation light).

The light source control means 37 is connected to the controller 59 in the processor and turns on or off the first and the second narrowband light sources 33, 34 according to an instruction by the controller 59.

The light source control means 37 is also connected to the illumination light amount correction means 38 to correct the illumination light amounts of the first and the second narrowband light N1, N2 emitted from the first and the second narrowband light sources 33, 34 based on the illumination light amount correction data from the illumination light amount correction means 38 and an instruction from the controller.

The correction of the illumination light amount is made in response to an instruction from the controller 59 so that the correction illumination light amounts determined by the illumination light amount correction means 38 are emitted from the first and the second narrowband light sources 33, 34, by, for example, controlling the current values allowed to flow by the light source control means 37 to the first and the second narrowband light sources 33, 34.

In response to an imaging instruction entered in the console 23, the console 23 transmits the instruction to the controller 59, which controls the light source control means 37, whereupon the first and the second narrowband light sources 33, 34 emit the first and the second narrowband light N1, N2, which pass through the the first and the second narrowband optical fibers 33a, 34a, the coupler 36, and the light guide 43 to achieve imaging.

As illustrated in Fig. 2, the endoscope 11 comprises a light guide 43, a CCD 44, an analog processing circuit (AFE: analog front end) 45, an imaging controller 46, and the scope characteristics storage means 42. The light guide 43 is a large-diameter optical fiber, a bundle fiber, or the like having its light-receiving end inserted in the coupler 36 in the light source device, whereas its light emitting end is provided with the phosphor 41, which is directed toward an illumination lens 48 located in the leading end portion 16a. The first and the second narrowband light N1, N2 emitted from the light source device 13 are guided through the light guide 43 before entering the phosphor 41 provided at the light emitting end.

Upon receiving the second narrowband light N2, the phosphor 41 produces white light, which is the broadband light BB, toward the illumination lens 48. The first narrowband light N1 is emitted as it is toward the illumination lens 48; the second narrowband light N2 is emitted in a given ratio toward the illumination lens 48

The first narrowband light N1 and the broadband light BB admitted in the illumination lens 48 pass through an illumination window 49 attached to the end face of the leading end portion 16a to illuminate the body cavity. The first narrowband light N1 and the broadband light BB reflected by the inside of the body cavity pass through an observation window 50 attached to the end face of the leading end portion 16a to enter a condenser lens 51.

The CCD 44 receives the light from the condenser lens 51 with its imaging surface 44a, performs photoelectric conversion of the received light to accumulate a signal charge, and reads out the accumulated signal charge as an imaging signal. The read-out imaging signal is transmitted to the AFE 45. The CCD 44 is a color CCD whose imaging surface 44a has arranged therein three colors of pixels, red pixels, green pixels, and blue pixels, each provided with one of a red filter, a green filter, and a blue filter.

The red filters, the green filters, and the blue filters have spectral transmittances 52, 53, and 54, as illustrated in Fig. 7, respectively. The broadband light BB entering the condenser lens 51 has a wavelength ranging from about 470 nm to 700 nm. Therefore, the red filters, the green filters, and the blue filters pass the broadband light BB having wavelengths respectively corresponding to their spectral transmittances 52, 53, 54. Now, let imaging signal R be a signal photoelectrically converted by a red pixel, imaging signal G a signal photoelectrically converted by a green pixel, and imaging signal B a signal photoelectrically converted by a blue pixel. Then, the broadband light BB entering the CCD 44 gives a broadband imaging signal composed of the imaging signal R, the imaging signal G, and the imaging signal B.

The first and the second narrowband light N1, N2 pass through the blue color filters, undergo photoelectric conversion by the blue pixels, yielding a first and a second narrowband imaging signal composed of the imaging signal B.

In the imaging signal B obtained according to this embodiment, the imaging signal B of the broadband imaging signal and the first and the second narrowband image signals are superposed.

The AFE 45 comprises a correlated double sampling circuit (CDS), an automatic gain control circuit (AGC), and an analog-to-digital converter (A/D) (none of them are shown). The CDS performs correlated double sampling of an imaging signal supplied from the CCD 44 to remove noise generated by actuation of the CCD 44. The AGC amplifies an imaging signal from which noise has been removed by the CDS. The analog-to-digital converter converts an imaging signal amplified by the AGC into a digital imaging signal having a given number of bits, which is applied to the processor 12.

The imaging controller 46 is connected to the controller 59 in the processor 12 and sends a drive signal to the CCD 44 in response to an instruction given by the controller 59. The CCD 44 outputs an imaging signal to the AFE 45 at a given frame rate according to the drive signal from the imaging controller 46. According to the first embodiment, a total of two operations are performed in one frame of acquisition period as illustrated in Fig. 8: a step of accumulating a signal charge through photoelectric conversion of the first narrowband light N1, the second narrowband light N2, and the broadband light BB and a step of reading out the accumulated signal charge as an imaging signal. These operations are repeated.

As illustrated in Fig. 2, the processor 12 comprises a digital signal processor 55 (DSP), a frame memory 56, a image producer 57, and a display control circuit 58, all of these components being controlled by the controller 59. The DSP 55 performs color separation, color interpolation, white balance adjustment, gamma correction, and the like of the imaging signals produced from the AFE 45 of the endoscope to produce image data. The frame memory 56 stores the image data produced by the DSP 55 and the scope characteristics data supplied from the scope characteristics storage means 42. The image data is color image data containing colors of red (R), green (G), and blue (B).

The image producer 57 contains a second reference table 61 representing the relationship between the wavelength shift amount and the variation amount in the pixel value in the image data obtained through photoelectric conversion by the CCD 44. The second reference table 61 can be produced by, for example, by emitting light by sequentially changing the light source wavelength by a given width to obtain the variation amount in the pixel values in the image data of the colors B, G, and R corresponding to the wavelength shift amount. The second reference table 61 may be stored in the scope characteristics storage means 42.

In addition to the second reference table 60, the scope characteristics storage means 42 stores the scope characteristics of the endoscope 11 obtained in the scope calibration.

The image producer 57 obtains the variation amount of the pixel value in the image data corresponding to the wavelength shift amount calculated by the wavelength shift amount calculation means referring to the second reference table and corrects the pixel values in the image data for a case where the light emitted from the light source device 13 is photoelectrically converted by the CCD 44 according to the obtained variation amount of the pixel value in the image data, i.e., so as to counteract the variation amount of the pixel value. Further, the image producer 57 corrects the gain in the photoelectric conversion by the CCD 44 so that the corrected pixel value of the image data agrees with the pixel value of the image data for the case where the light having the reference wavelength is photoelectrically converted by the CCD 44.

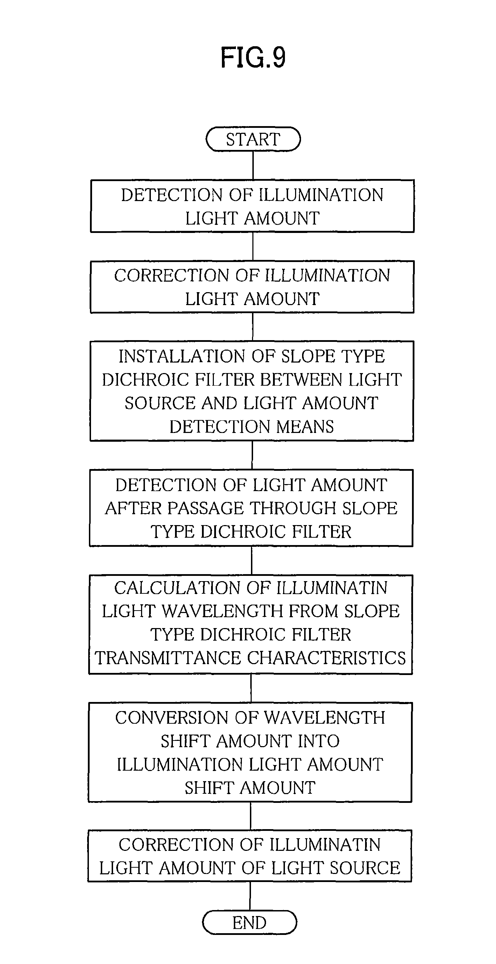

Next, the calibration of the light source device 13 will be described referring to the flowchart illustrated in Fig. 9.

The console 23 or an input means, not shown, provided in the light source device 13 are used to operate the light source control means 37 with the slope type dichroic filter 25 removed to emit narrowband light MO from the narrowband light source.

The emission of the narrowband light is sequentially carried out using the first and the second narrowband light sources 33, 34. Similar calibration is carried out with the first and the second narrowband light sources 33, 34.

Description will be continued below taking the first narrowband light source 33 as an example. Upon emission of the narrowband light M01, the light amount detection means 26 detects a light amount L01 of the emitted narrowband light M01, and the information on the light amount L01 is sent to the wavelength detection means 35 and the illumination light amount correction means 38.

The illumination light amount correction means 38 receives the illumination light amount L01 of the narrowband light M01 and obtains the light amount difference between the M01 and a reference illumination light amount L11 to send to the light source control means 37 illumination light amount correction data for making the light amount difference zero.

The light source control means 37 corrects the illumination light amount of the narrowband light source 33 so that the illumination light amount detected by the light amount detection means 26 is equal to the reference illumination light amount L11.

Thus, the illumination light amount of the light emitted from the narrowband light source 33 is adjusted to the reference illumination light amount L11.

Next, with the slope type dichroic filter 25 corresponding to the first narrowband light N1 installed, the light source control means 37 is operated to cause the first narrowband light source 33, which is now adjusted to the reference light amount L11, to emit the narrowband light M11.

The narrowband light M11 passes through the slope type dichroic filter 25, whereupon the light amount detection means 26 detects an illumination light amount L21 after transmission, sending the information thus obtained to the wavelength detection means 35.

The wavelength detection means 35 acquires information on the transmittance characteristics of the slope type dichroic filter 25 from the light source characteristics storage means 40 and calculates a wavelength W11 of the narrowband light M11 from the ratio between the reference light amount L11 and the transmitted illumination light amount L21 and the transmittance characteristics of the slope type dichroic filter 25.

Then the wavelength shift amount calculation means 39 calculates the wavelength shift amount, which is the difference between a wavelength detected by the wavelength detection means 35 and the preset reference wavelength W01. The calculated wavelength shift amount is sent to the illumination light amount correction means 38 as wavelength shift amount data.

For the second narrowband light source 34, the wavelength shift amount calculation means 39 converts the wavelength shift amount into an illumination light amount shift amount by addition of the fluorescence characteristics of the phosphor.

The conversion of the wavelength shift amount into the illumination light amount shift amount is achieved as described above by obtaining the effects of the reflectance and the spectral sensitivity on the wavelength shift amount based on Fig. 6 and other figures and correcting the illumination light correction amount so as to counteract the effects.

The illumination light amount correction means 38 corrects the light amount of the first and the second narrowband light N1, N2 emitted from the light source device 13 according to the wavelength shift amount calculated by the wavelength shift amount calculation means 39, that is, so as to counteract the effects by the wavelength shift amount.

The illumination light amount correction means 38 may also adjust the wavelength of the first narrowband light N1 to the reference wavelength by, for example, adjusting the temperature of the first narrowband light source (laser light source) 33 according to the illumination light amount shift amount.

The reference wavelength W01 and the reference light amount L11, the wavelength shift amount and the illumination light amount shift amount, and the wavelength W11 and an illumination light amount L31 of the first narrowband light source are stored in the light source characteristics storage means 40.

Thus, the first narrowband light source 33 is calibrated in the same manner as if illumination light having the reference wavelength and the reference light amount is emitted to the reference endoscope comprising the reference phosphor.

Calibration is similarly carried out also for the second narrowband light source 34, and the reference wavelength W02 and the reference light amount L12, the wavelength shift amount and the illumination light amount shift amount, and the wavelength W12 and the illumination light amount L32 of the second narrowband light source are stored in the light source characteristics storage means 40.

The calibration of the light source means 13 is carried out as described above.

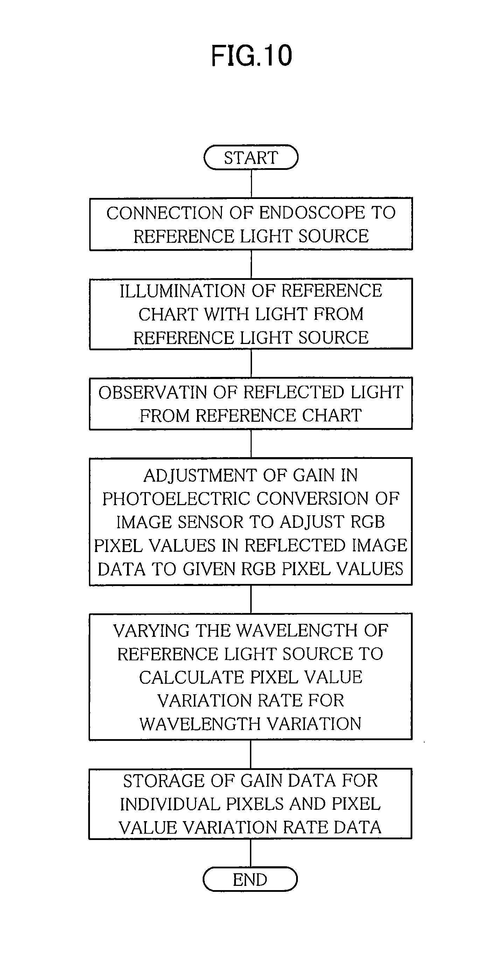

Next, the calibration of the endoscope 11 will be described referring to the flowchart illustrated in Fig. 10.

First, the endoscope 11 is connected to the reference light source that emits the reference light amount with the reference wavelength and supplied with the reference light emitted from the reference light source, whereupon the light from the reference light source passes through the phosphor 41 provided at the leading end portion 16a of the endoscope to illuminate the reference chart with broadband light and narrowband light.

The reference light source is an ideal light source for emitting light having a given wavelength in a given light amount.

The reference chart shows a pattern of outputs of all the pixels of the image data as pixel values of image data.

The light irradiating the reference chart is reflected by the reference chart, and the reflected light is received by the imaging surface 44a of the CCD 44, an image sensor, provided at the leading end portion 16a of the endoscope.

The reflected light received by the CCD 44 is sent to the image producer 57 as reflected image data through the AFE 45, DSP 55, and the frame memory 56.

The reflected image data is compared in the image producer 57 with reference image data previously provided in the image producer 57 (image data of pixel values corresponding to the reference chart) to adjust the gain in photoelectric conversion by the CCD 44 so that the RGB pixel values of the reflected image data agree with the RGB pixel values of the reference image data.

After the pixel gain adjustment, the wavelength of the reference light source is varied to calculate the pixel value variation rate of the reflected image data corresponding to the wavelength variation of the reference light to produce a second reference table 61.

The reference image data, adjusted gain data of the individual pixels, and the pixel value variation rate data corresponding to the wavelength variation are recorded in the scope characteristics storage means 42 as scope characteristics of the endoscope 11.

The calibration of the endoscope 11 is carried out as described above.

Suppose that the light source device 13 and the endoscope 11 have been calibrated with each other and that the reference endoscope is installed, the light source device 13 can, upon emission of the first and the second narrowband light N1, N2, emit given first and second narrowband light N1, N2 and given broadband light BB from the leading end portion 16a of the reference endoscope as described above, while the endoscope 11 can, when provided with the reference light source, emit given first and second narrowband light N1, N2 and given broadband light BB from the leading end portion 16a of the endoscope to acquire a given reflected image.

The above applies only when the reference light source or the reference endoscope is provided. In the market, because of the variations in the characteristics of the light source device 13 and the endoscope 11, desired characteristics cannot be obtained even when the reference light source and the reference endoscope connected have previously been calibrated, thus necessitating calibration of the endoscope system comprising the light source device 13 and the endoscope 11 after installation in order to obtain the endoscope system 10 having desired characteristics.

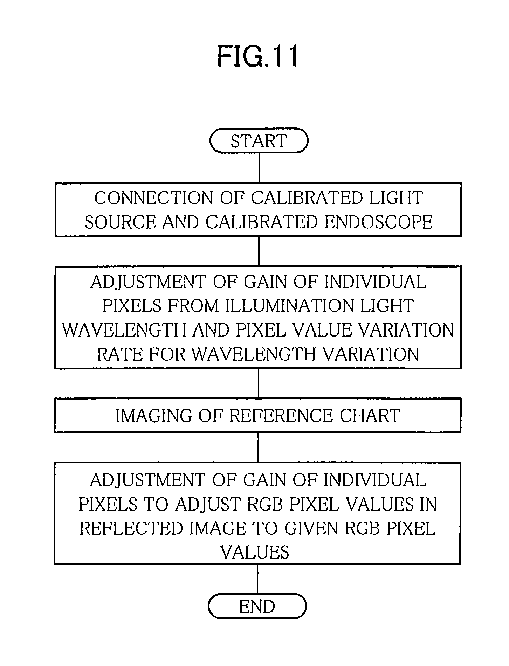

Therefore, we will describe a method of calibration for a case where in the market the endoscope 11 is attached to the light source device 13 referring to the flowchart illustrated in Fig. 11.

First, the endoscope 11 having stored therein scope characteristics data is connected to a calibrated light source device 13.

Upon connection of the light source device 13 and the endoscope 11, the wavelength shift amount of the light source that was calculated in the calibration of the light source device 13 and stored in the light source characteristics storage means 40 is read out to the image producer 57 through the frame memory.

The image producer 57 calculates a gain correction value of the image sensor from the wavelength shift amount of the light source and the pixel value variation rate data for the wavelength variation that was calculated in the calibration of the endoscope 11 and stored in the scope characteristics storage means.

Incorporating the gain correction value in the gain of the image sensor permits counteracting the effects of the wavelength shift of the light emitted from the light source device 13.

Finally, in this configuration, the reference chart is imaged to adjust the pixel gain of the individual pixels so that the RGB pixel values obtained from the reflected image data agree with the RGB pixel values of the reference pixel data.

The adjustment is made of the light amount of the illumination light. The wavelength shift amount of the laser light, which changes according to the operating current value of the laser light source, is preferably also corrected at the same time as the light amount is adjusted.

The calibration of the endoscope 10 is carried out in the market as described above.

As described above, calibration of the light source device 13 and the endoscope 11 followed by calibration made in the market of the light source device 13 and the endoscope 11 connected to each other ensures that the endoscope system 10 enables acquisition of substantially the same image with any light source device and any endoscope connected as when the reference light source device and the reference endoscope are connected regardless of the light source characteristics of the light source device 13 or the transmission characteristics of the endoscope 11 and the fluorescence characteristics of the phosphor 41.

The calibration of the endoscope system of the invention is carried out as described above.

Note that the reflectance and the spectral sensitivity of the illumination light for the wavelength shift amount of the illumination light vary with the biological site and the disease to be imaged.

The illumination light amount correction means 38 preferably contains a plurality of first reference tables 60 each representing the relationship between the wavelength of the light and the integrated value of the sensitivity of the image sensor and the reflectance of the reflected light for individual sites or diseases imaged by the electronic endoscope. In this case, the illumination light amount correction means 38 selects the first reference table 60 according to the site or disease to be imaged by the electronic endoscope, obtains the integrated value corresponding to the wavelength of the light emitted from the light source device referring to the selected first reference table 60, and changes the light amount correction amount according to the obtained integrated value.

The present invention is basically as described above. The present invention is not limited to any of the embodiments described above and permits various modifications to be made without departing from the spirit of the present invention.

## Claims

1. An electronic endoscope system, comprising:
a light source device for emitting light having a given wavelength band;
an electronic endoscope comprising a phosphor for producing white light with which a subject tissue in a body cavity is illuminated by causing the emitted light from said light source device to hit said phosphor as excitation light, and an image sensor for outputting image data of an image obtained by photoelectric conversion of reflected light of the white light illuminating the subject tissue;
wavelength detection means for detecting a wavelength of the emitted light from said light source device;
wavelength shift amount calculation means for calculating a wavelength shift amount, which is a difference between a wavelength detected by said wavelength detection means and a preset reference wavelength; and
first correction means for correcting a light amount of the emitted light from the light source device according to the wavelength shift amount calculated by said wavelength shift amount calculation means and second correction means for correcting a gain in the photoelectric conversion by said image sensor.

2. The electronic endoscope system according to Claim 1, wherein said first correction means comprises a first reference table representing a relationship between a wavelength of light and an integrated value of a sensitivity of said image sensor and a reflectance of the reflected light, obtains an integrated value corresponding to a wavelength of the emitted light from said light source device referring to the first reference table, and changes the light amount correction amount according to the obtained integrated value.

3. The electronic endoscope system according to Claim 1, wherein said first correction means comprises plural first reference tables each representing a relationship between a wavelength of light and an integrated value of a sensitivity of said image sensor and a reflectance of the reflected light for each site or disease imaged by said electronic endoscope, selects one first reference table from the plural first reference tables according to the site or disease to be imaged by said electronic endoscope, obtains an integrated value corresponding to a wavelength of the emitted light from the light source device referring to the selected one first reference table, and changes the light amount correction amount according to the obtained integrated value.

4. The electronic endoscope system according to any one of Claims 1 to 3,
wherein said light source device is a laser light source for emitting laser light, and
wherein said wavelength shift amount calculation means calculates the wavelength shift amount by further adding thereto a wavelength shift amount of the laser light that changes according to an operating current value of said laser light source.

5. The electronic endoscope system according to any one of Claims 1 to 4, wherein said wavelength detection means obtains a transmittance being a ratio between a first light amount and a second light amount, from the first light amount of the emitted light from said light source device and the second light amount of the emitted light from said light source device and passed through a slope type dichroic filter whose transmittance changes for light having a given wavelength in proportion to the wavelength of the light, and detects the wavelength of the emitted light from said light source device according to the obtained transmittance.

6. The electronic endoscope system according to any one of Claims 1 to 5, wherein said second correction means comprises a second reference table representing a relationship between a wavelength shift amount and a variation amount in a pixel value in the image data obtained through the photoelectric conversion by said image sensor, obtains the variation amount of the pixel value in the image data corresponding to the wavelength shift amount calculated by the wavelength shift amount calculation means referring to the second reference table, corrects a pixel value in the image data for a case where the emitted light from said light source device is photoelectrically converted according to the obtained variation amount of the pixel value in the image data, and further corrects the gain in the photoelectric conversion by said image sensor so that the corrected pixel value of the image data agrees with a pixel value in image data for a case where the light having the reference wavelength is photoelectrically converted.

7. A method of calibrating an electronic endoscope provided with a light source device for emitting light having a given wavelength band and an image sensor that illuminates a subject tissue in a body cavity with white light produced by causing excitation light emitted from the light source device to hit a phosphor and outputs image data obtained by photoelectric conversion of reflected light of the white light illuminating the subject tissue, the method comprising:
a wavelength detection step of detecting a wavelength of light emitted from the light source device;
a wavelength shift amount detection step of detecting a wavelength shift amount, which is a difference between the detected wavelength and a preset reference wavelength;
a first correction step of correcting a light amount of the light emitted from the light source device according to the calculated wavelength shift amount; and
a second correction step of correcting a gain in photoelectric conversion by the image sensor according to the calculated wavelength shift amount.

8. The calibration method according to Claim 7, wherein said first correction step comprises obtaining an integrated value corresponding to a wavelength of the emitted light from said light source device referring to a first reference table representing a relationship between a wavelength of light and an integrated value of a sensitivity of said image sensor and a reflectance of the reflected light, and changing the light amount correction amount according to the obtained integrated value.

9. The calibration method according to Claim 7, wherein the first correction step comprises selecting one first reference table according to a site or disease to be imaged by said electronic endoscope from plural first reference tables each representing a relationship between a wavelength of light and an integrated value of a sensitivity of said image sensor and a reflectance of the reflected light for each site or disease imaged by said electronic endoscope, obtaining an integrated value corresponding to a wavelength of the emitted light from the light source device referring to the selected one first reference table, and changing the light amount correction amount according to the obtained integrated value.

10. The calibration method according to any one of Claims 7 to 9,
wherein said light source device is a laser light source for emitting laser light, and
wherein said wavelength shift amount calculation step calculates the wavelength shift amount by further adding thereto a wavelength shift amount of the laser light that changes according to an operating current value of said laser light source.

11. The calibration method according to any one of Claims 7 to 10,
further comprising: a detecting step of detecting a first light amount of the emitted light from said light source device and a second light amount of the emitted light from said light source device and passed through a slope type dichroic filter whose transmittance changes for light having a given wavelength in proportion to the wavelength of the light,
wherein said wavelength detection step comprising obtaining a transmittance being a ratio between the first light amount and the second light amount, from the obtained first and the second light amounts, and detecting the wavelength of the emitted light from said light source device according to the obtained transmittance.

12. The calibration method according to any one of Claims 7 to 11,
wherein said second correction step comprises obtaining a variation amount of a pixel value in image data corresponding to the calculated wavelength shift amount referring to a second reference table representing a relationship between a wavelength shift amount and a variation amount in a pixel value in the image data obtained through the photoelectric conversion by said image sensor, correcting a pixel value in the image data for a case where the emitted light from the light source device is photoelectrically converted according to the obtained variation amount of the pixel value in the image data, and further correcting the gain in the photoelectric conversion by the image sensor so that the corrected pixel value of the image data agrees with a pixel value of the image data for a case where the light having the reference wavelength is photoelectrically converted.

13. The calibration method according to any one of Claims 7 to 12,
further comprising: a step of illuminating a reference chart with light having the reference wavelength, and a step of obtaining the image data outputted from said image sensor after the photoelectric conversion of the reflected light from the reference chart by said image sensor,
wherein said second correction step comprises adjusting the gain in the photoelectric conversion by said image sensor so that a pixel value in the image data outputted by said image sensor after the photoelectric conversion of the reflected light from the reference chart agrees with a reference value corresponding to the reference chart.
